# EUROPEAN PATENT APPLICATION

(11) **EP 2 452 617 A1**
(43) Date of publication of application: **16.05.2012**
(21) Application number: 11188315.3
(22) Date of filing: 08.11.2011
(51) Int. Cl.: A61B 5/00, A61B 5/04

(54) **System and method for wireless transmission of neural data**

(30) Priority: 11.11.2010 US 412661 P
(71) Applicant: I2S Micro Implantable Systems, LLC, Salt Lake City, UT 84108 (US)
(72) Inventor: Sorenson, Michael, Salt Lake City, UT 84108 (US); Decaria, Christine, Salt Lake City, UT 84105 (US)
(74) Representative: Zardi, Marco

(57) **Abstract**

Embodiments of the invention generally relate to a neuralphysiological data acquisition system configured to wirelessly transmit neural data from a patient to a receive subsystem. In an embodiment, a neuralphysiological data acquisition system includes a plurality of electrodes, a headstage, and a wireless module. The electrodes are configured to be implanted subcutaneously within neural tissue of a patient and to collect analog neural data from the patient. The headstage is coupled to the electrodes, and configured to receive and convert the analog neural signals to digital output. The wireless module is coupled to the headstage and configured to wireless transmit a signal representing the digital output to a receive subsystem including a wireless receiver.

## Description

### CROSS-REFERENCE TO RELATED APPLICATIONS

This application claims the benefit of U.S. Provisional Patent Application No. 61/412,661 filed on 11 November 2010 entitled "SYSTEM AND METHOD FOR WIRELESS TRANSMISSION OF NEURAL DATA," which is incorporated herein, in its entirety by this reference.

### BACKGROUND

Neuralphysiological data acquisition systems are configured to record and analyze animal or human brain and/or peripheral-nerve electrical activity. Such systems typically include one or more sensors that detect neural signals indicative of the brain or peripheral-nerve electrical activity near the sensor. Neural signals detected by the sensors can be collected and processed to assist in the study and/or treatment of, for example, sensory perception, motor control, learning and memory, attention, cognition and decision making, drug and toxin effects, epilepsy, Parkinson's, neuroprosthetics, brain-machine interfaces, neurostimulation therapies, dystonia, traumatic brain injury, and stroke.

While there are a variety of different available neuralphysiological data acquisition systems, manufacturers and users of such systems continue to seek improved system designs that provide effective data acquisition.

### SUMMARY

Embodiments of the invention generally relate to a neuralphysiological data acquisition system configured to wirelessly transmit neural data from a patient to a receive subsystem. In an embodiment, a neuralphysiological data acquisition system includes a plurality of electrodes, a headstage, and a wireless module. The electrodes are configured to be implanted subcutaneously within neural tissue of a patient and to collect analog neural data from the patient. The headstage is coupled to the electrodes, and configured to receive and convert the analog neural signals to digital output. The wireless module is coupled to the headstage and configured to wireless transmit a signal representing the digital output to a receive subsystem including a wireless receiver.

Alternately or additionally, embodiments include a wireless module that may be implemented in a neuralphysiological data acquisition system. Various embodiments for wireless modules are provided below.

In an embodiment, a method of collecting and conditioning neural signals includes collecting analog neural signals from neural tissue of a patient. The method additionally includes conditioning the collected analog neural signals at the patient to generate a digital output representing the collected analog neural signals. The method additionally includes wirelessly transmitting a signal representing the digital output from the patient to a receive subsystem including a wireless receiver.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the invention, wherein identical reference numerals refer to identical elements or features in different views or embodiments shown in the drawings.

FIG. 1 is a schematic diagram of one example of an operating environment for the disclosed neuralphysiological data acquisition system embodiments.

FIG. 2 is a functional block diagram of an embodiment of a collection, conditioning, and transmission subsystem that may be implemented in the example operating environment 100 of FIG. 1.

FIG. 3A is a cross-sectional view of an embodiment of a collection, conditioning, and transmission subsystem including both internally implanted components and externally mounted components such as may be implemented in the example operating environment of FIG. 1.

FIG. 3B is a cross-sectional view of another embodiment of a collection, conditioning, and transmission subsystem including primarily internally implanted components such as may be implemented in the example operating environment of FIG. 1.

FIG. 4A is a cross-sectional view of an implantable component that generically represents an implantable headstage, an implantable wireless module, or other implantable component that may be implemented in one or more of the subsystems of FIGS. 1-3B according to some embodiments.

FIG. 4B is a cross-sectional view of an example of another implantable component that generically represents an implantable headstage, an implantable wireless module, or other implantable component that may be implemented in one or more of the subsystems of FIGS. 1-3B.

FIG. 5 is a functional block diagram of an embodiment of a wireless module that may be implemented in the subsystems of FIGS. 1-3B.

FIG. 6 is a flowchart of an embodiment of a method of collecting and conditioning analog neural signals.

### DETAILED DESCRIPTION

Embodiments of the invention generally relate to a neuralphysiological data acquisition system configured to wirelessly transmit neural data from a patient to a receive subsystem.

### I. EXAMPLE OPERATING ENVIRONMENT

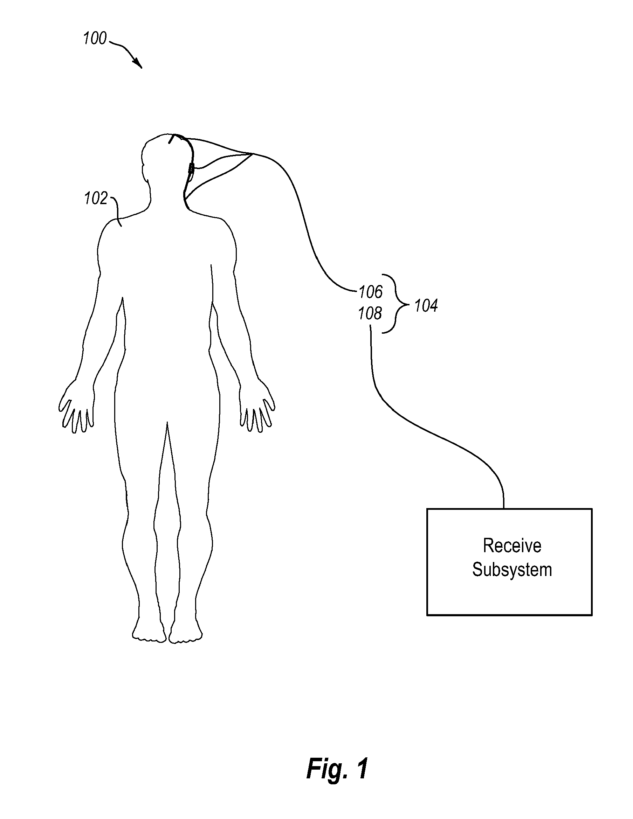

One example operating environment 100 is illustrated in FIG. 1 for the disclosed neuralphysiological data acquisition system embodiment. The example operating environment 100 includes a patient or test subject 102 (hereinafter "patient 102") and a neuralphysiological data acquisition system 104 (hereinafter "system 104"), including collection, conditioning and transmission subsystem 106 (hereinafter "subsystem 106") and a receive subsystem 108.

The subsystem 106 is generally configured to collect, condition, and wirelessly transmit neural data to the receive subsystem 108. In some embodiments, power consumption of the subsystem 106 may be about four watts. More generally, the power consumption of the subsystem 106 may be between about 0.5 watts and about 5 watts depending on the configuration of subsystem 106.

The receive subsystem 108 is generally configured to wirelessly receive the transmitted neural data and perform some action on, or in response to, the neural data. Accordingly, the receive subsystem 108 typically includes a wireless receiver. While FIG. 1 illustrates the receive subsystem 108 as being external to the patient 102, in other embodiments, the receive subsystem 108 may be implanted (in whole or in part) within the patient 102, or is mechanically coupled to the patient 102 (e.g., being worn by the patient).

In the illustrated embodiment of FIG. 1, the patient 102 is a human patient. In other embodiments, the patient 102 may be an animal test subject, such as an avian, rodent, feline, or primate patient, or other suitable test subject or patient.

The system 104 is generally configured to collect, condition, and wirelessly transmit data representing brain and/or peripheral nerve activity of the patient 102. Consistent with the foregoing, the subsystem 106 may be configured to collect analog neural signals output by the patient 102. In addition, the subsystem 106 may be configured to amplify, multiplex and digitize and otherwise condition the collected analog neural signals to generate a digital output. Alternately or additionally, the subsystem 106 may be configured to wirelessly transmit a signal representing the digital output to the receive subsystem 108. Additional details of the subsystem 106 and system 104 are provided below.

The receive subsystem 108 is generically representative of any device configured to receive wirelessly transmitted neural data from the subsystem 106 and to perform some action on, or in response to, the neural data. Generally, the receive subsystem 108 includes a wireless receiver(s) configured to receive a wireless signal(s) representing the neural data and to convert the wireless signal(s) to an electrical signal(s) representing the neural data.

In some embodiments, the receive subsystem 108 is an external processing system configured to process the neural data for analyzing the brain and/or peripheral nerve activity of the patient 102. For example, the external processing system may be configured to extract information about a brain function of the patient 102 from the signal representing the digital output. The extracted information may relate to or identify anomalous brain functions such as may be associated with seizures or other anomalies, or normal brain functions such as may be associated with controlling a biological function or a portion of the patient's 102 body, or other known or unknown brain functions.

In these and other embodiments, the receive subsystem 108 may include, in addition to a wireless receiver, one or more of a digital-to-analog converter ("DAC"), a front-end amplifier, a neural signal processor ("NSP"), or a general purpose or special purpose computer. Aspects of various external processing systems and components thereof that may be suitable for implementation in the receive subsystem 108 according to some embodiments are disclosed in United States Patent Application No. 12/906,792 entitled IMPLANTABLE NEURAL SIGNAL ACQUISITION APPARATUS, filed October 18, 2010, the disclosure of which is incorporated herein, in its entirety, by this reference.

Alternately or additionally, the receive subsystem 108 may include, in addition to a wireless receiver, a drug delivery device, such as an electronically controlled intravenous ("IV") drip, or the like. In these and other embodiments, the drug delivery device may be configured to deliver, intravenously or otherwise, a drug to the patient 102 in response to identifying a predetermined pattern in the wireless signal received from the subsystem 106. The predetermined pattern may be indicative of an oncoming biological event in the patient 102 that may be preventable by or the severity of which may be reduced by the drug. For example, the predetermined pattern may be a pattern indicative of an oncoming seizure in the patient where the drug can prevent or reduce the severity of the oncoming seizure if delivered to the patient soon after identifying the predetermined pattern in the signal received from the subsystem 106.

Alternately or additionally, the receive subsystem 108 may include, in addition to a wireless receiver, an electro-stimulation device. In these and other embodiments, the electro-stimulation device may be configured to apply an electric current to the patient's 102 brain in response to identifying a predetermined pattern in the wireless signal received from the subsystem 106. The predetermined pattern may be indicative of an oncoming biological event in the patient 102, such as a seizure. Application of the electric current to the patient's brain 102 may be configured to prevent or reduce the severity of the oncoming biological event.

Alternately or additionally, the receive subsystem 108 may include, in addition to a wireless receiver, a notification device. In these and other embodiments, the notification device may be configured to generate an alert in response to identifying a predetermined pattern in the wireless signal received from the subsystem 106. The predetermined pattern may be indicative of an oncoming biological event in the patient 102, such as a seizure. Thus, if the patient 102 is driving a car or is engaged in some other activity during which impaired motor control associated with a seizure could result in harm to the patient 102, the patient 102 can pull over or otherwise prepare for the oncoming seizure after receiving the alert from the notification device.

Alternately or additionally, the receive subsystem 108 may include, in addition to a wireless receiver, a data storage device. In these and other embodiments, the data storage device may be configured to store data representing the wireless signal, and thus the neural data, received from the subsystem 106. Optionally, the stored data may be subsequently provided to an external processing system such as disclosed in the 12/906,792 patent application which was previously incorporated by reference to analyze the brain and/or peripheral nerve activity of the patient. The data storage device may optionally be portable to enable neural data of the patient 102 to be acquired in, for example, non-clinical settings. For instance, a portable data storage device may enable neural data to be acquired while the patient 102 is carrying out everyday tasks in familiar environments such as at home or work.

In this regard, the present disclosure appreciates that neural data acquisition for epileptic patients is often performed in hospitals or other clinical environments that are relatively unfamiliar to the patients. Removal of the patients from their familiar, everyday environments can complicate neural data acquisition as it may eliminate stimuli that cause seizures or otherwise result in a change in the frequency, severity or duration of the seizures experienced by the patients. To induce seizures in patients so that relevant neural data can be acquired while patients are in the hospital or other clinical setting, the patients are often subjected to sleep deprivation, alterations in medications, or other seizure-inducing therapies.

According to some embodiments in which the receive subsystem 108 includes a portable data storage device, however, neural data can be acquired while the patient carries out everyday tasks in familiar environments. By acquiring neural signal data while the patient is carrying out everyday tasks in familiar environments and recording the acquired signal data in the portable data storage device of receive subsystem 108, there is no change in the patient's 102 environment that might otherwise result in a change in, e.g., the frequency, severity or duration of the seizures typically experienced by the patient 102. Accordingly, sleep deprivation, medication alteration, and other seizure-inducing therapies to which patients in clinical settings are often subjected to induce seizures and acquire relevant neural data can be reduced or completely eliminated according to some embodiments while still allowing relevant neural data to be acquired.

Many of the embodiments described above are disclosed in the context of a patient 102 having epilepsy or other condition that results in seizures. However, the embodiments disclosed above are provided by way of example only and are not intended to limit the scope of the present disclosure. In particular, the embodiments disclosed herein are not limited to use in patients 102 having epilepsy or other condition that results in seizures. Indeed, the embodiments already described above can be used for delivering drugs to a patient 102, generating an alert for a patient 102, storing data representing neural activity of a patient, or the like, in patients with conditions other than epilepsy or without any particular or known condition at all. Alternately or additionally, some embodiments may be implemented to control prosthetic limbs (e.g., in amputee patients 102) or voice synthesizers (e.g., in certain patients 102 that are unable to speak), or particular biological functions performed by portions of the patient's 102 body, or the like or any combination thereof

As an example, the receive subsystem 108 may include, in addition to a wireless receiver, a prosthetic limb. In these and other embodiments, the prosthetic limb may be configured to operate in accordance with the wireless signal received from the subsystem 106. For example, the subsystem 106 may be configured to collect and transmit to the receive subsystem 108, including prosthetic limb, neural signals representing limb-control signals generated by the patient's 102 brain. A processor included in the receive subsystem 108 or the subsystem 106 may be configured to convert the neural signals to control signals that can be used to operate the prosthetic limb.

Alternately or additionally, the receive subsystem 108 may include, in addition to a wireless receiver, a voice synthesizer. In these and other embodiments, the voice synthesizer may be configured to synthesize speech corresponding to a word or words represented by the wireless signal, and thus the neural data, received from the subsystem 106. Thus, if the patient 102 is unable to speak, neural signals generated by the patient's 102 brain that are indicative of words can be acquired by the subsystem 106 and transmitted to the voice synthesizer, whereupon the voice synthesizer may synthesize speech corresponding to the words.

The foregoing embodiments of the receive subsystem 108 are merely representative of the receive subsystem 108 and should not be construed to limit the scope of the present disclosure.

### II. COLLECTION, CONDITIONING AND TRANSMISSION SUBSYSTEM

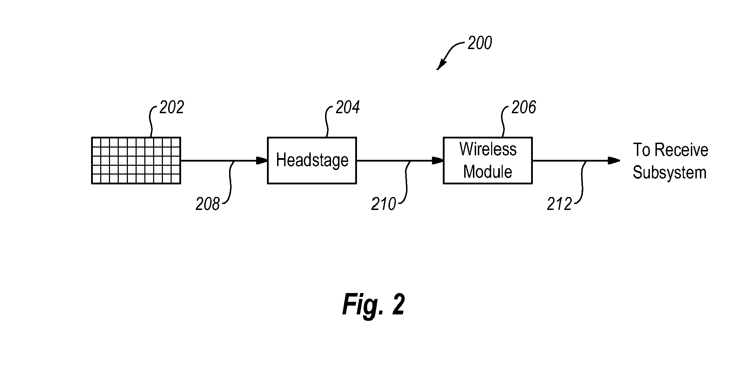

FIG. 2 is a functional block diagram of a collection, conditioning, and transmission subsystem 200 (hereinafter "subsystem 200") such as may be implemented in the example operating environment 100 of FIG. 1. The subsystem 200 may correspond to the subsystem 106 of FIG. 1. In the illustrated embodiment of FIG. 2, the subsystem 200 includes a plurality of electrodes 202, a headstage 204, and a wireless module 206.

The electrodes 202 are configured, in some embodiments, to be subcutaneously implanted within neural tissue, such as cortical tissue or peripheral nerve tissue, of a patient and to collect analog neural signals 208 from the neural tissue of the patient. Each electrode 202 serves as a neural interface that essentially connects neurons to electronic circuitry. The electrodes 202 may include multiple implantable individual stiff-wire electrodes, an implantable microelectrode or microwire array, planar silicon probes, a subdural electrocorticography ("ECoG") grid, epidural electroencephalography ("EEG") electrodes, or other suitable implantable electrodes or electrode arrangement.

The headstage 204 is electrically coupled to the electrodes 202 and is generally configured to receive and convert the analog neural signals 208 to digital output 210. More generally, the headstage 204 is configured to condition the analog neural signals 208 to generate digital output 210. According to some embodiments, conditioning the analog neural signals 208 may include amplifying the analog neural signals 208, filtering the amplified analog neural signals, multiplexing the filtered analog neural signals to generate multiplexed analog neural signals, digitizing the multiplexed analog neural signals, packetizing the digital neural signals for inclusion in the digital output 210, or combinations of the foregoing.

The headstage 204 may be configured to be mounted external to the patient. Alternately or additionally, the headstage 204 may be configured to be implanted within a patient as an implantable headstage. Whether externally mounted or implanted within a patient, the headstage 204 is generally located within a relatively short transmission distance from the electrodes 202. Transmission distance refers to the distance a signal travels along a corresponding signal medium between a signal source and a receiver, as opposed to the direct physical distance between the source and the receiver. While the transmission distance between the electrodes 202 and headstage 204 may in some cases be about equal to the direct physical distance, in many cases the transmission distance is greater than the physical distance.

In some embodiments, the transmission distance between electrodes 202 and headstage 204 may range from about 1.5 cm to about 30 cm, or from about 5 cm to about 24 cm. In other embodiments, the headstage 204 may be mounted directly to one or more of the electrodes 202 or integrated into one or more of the electrodes 202. In these and other embodiments, the transmission distance between electrodes 202 and headstage 204 may be less than 1.5 centimeters. In still other embodiments, the transmission distance between electrodes 202 and headstage 204 may be greater than 30 cm.

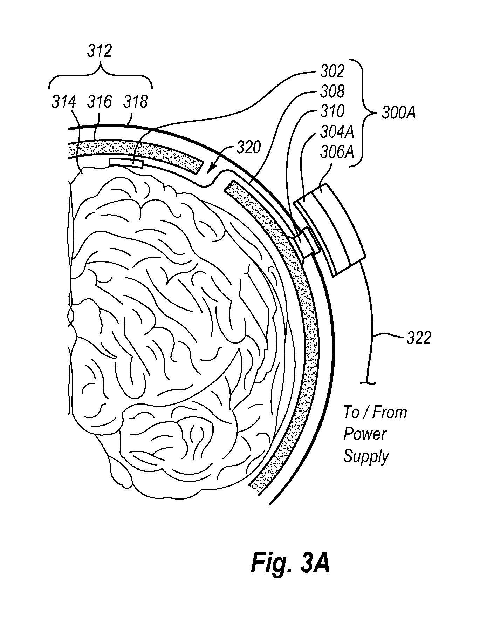

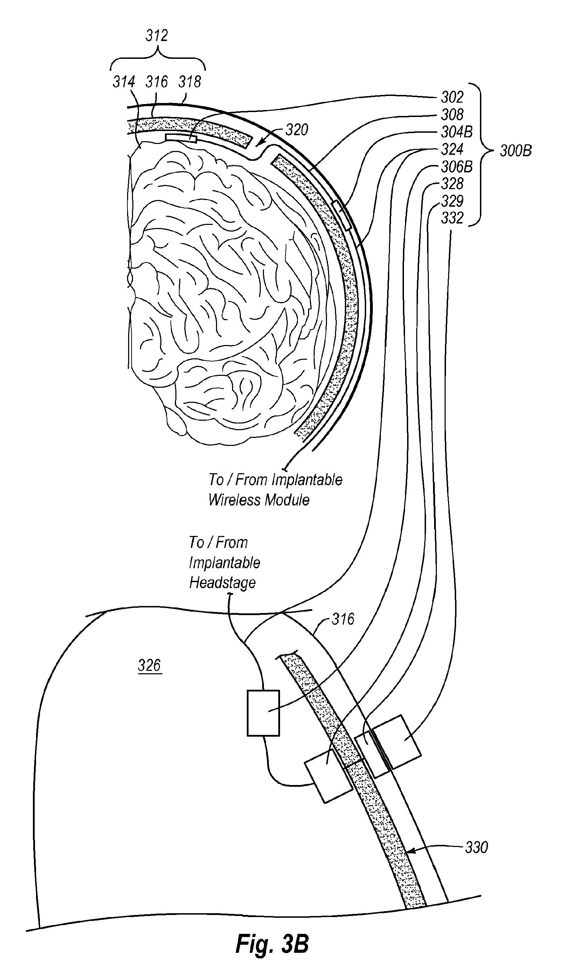

Various headstages that may be suitable for implementation in the subsystem 200 according to some embodiments are described as implantable electronics package(s) in United States Patent Application No. 12/906,792, which was previously incorporated by reference. Although many of the implantable electronics packages disclosed in United States Patent Application No. 12/906,792 are implantable in a test subject, it is not necessary that the headstages described herein be implantable, or that the implantable headstages disclosed herein be implanted in a patient when used. Indeed, certain embodiments disclosed herein include implantable headstages (see, e.g., FIG. 3B), while other embodiments disclosed herein include headstages, whether implantable or non-implantable, that are not implanted in a patient during use (see, e.g., FIG. 3A).

With continued reference to FIG. 2, the wireless module 206 is electrically coupled to the headstage 204 and is generally configured to wirelessly transmit a wireless signal 212 representing the digital output 210 to a receive subsystem, such as the receive subsystem 108 of FIG. 1. The wireless signal 212 may include, for example, a radio frequency ("RF") carrier, an infrared ("IR") carrier, or other suitable wireless carrier having a different wavelength(s). Alternately or additionally, the wireless module 206 may be configured to implement any one of a variety of wireless protocols, including, but not limited to, the 802.11n protocol, the Bluetooth protocol, or the like, or variations thereof. For example, according to some embodiments, the wireless module 206 implements a modified version of the 802.11n protocol, as explained in greater detail below.

The wireless module 206 may be configured to be mounted external to the patient. Alternately or additionally, the wireless module 206 may be configured to be implanted within a patient as an implantable wireless module. In externally mounted embodiments, the wireless module 206 may be mounted directly to, for example, the headstage 204. In implantable embodiments, the wireless module 206 may be mounted directly to the headstage 204 or may be positioned in a separate location from the headstage 204. For instance, the headstage 204 may be implanted subcutaneously on or near a patient's skull, while the wireless module 206 may be implanted within the patient's chest cavity or other location somewhat remote from the headstage 204. Of course, wires or other electrically conductive elements may be routed through the patient's body to electrically couple the wireless module 206 to the headstage 204.

### A. Internal/External Implementation

FIG. 3A illustrates an embodiment of a collection, conditioning, and transmission subsystem 300A (hereinafter "subsystem 300A") including both internally implanted components and externally mounted components such as may be implemented in the example operating environment 100 of FIG. 1 according to some embodiments. The subsystem 300A may correspond to the subsystems 106, 200 of FIGS. 1 and 2. Similar to the subsystem 200 of FIG. 2, the subsystem 300A of FIG. 3A includes a plurality of electrodes 302, a headstage 304A, and a wireless module 306A.

FIG. 3A additionally illustrates a patient 312 including cortical tissue 314, cranium 316, and skin 318. A hole 320 drilled in the cranium 316 permits the electrodes 302 to be implanted subcutaneously within the cortical tissue 314. More particularly, the electrodes 302 are implanted subcranially in FIG. 3A. Although the electrodes 302 are shown as being disposed on the surface of the cortical tissue 314 between the cranium 316 and the cortical tissue 314, the electrodes 302 may alternately or additionally penetrate into the cortical tissue 314.

Additionally, the subsystem 300A includes a wire bundle 308 and a pedestal 310. The wire bundle 308 is coupled between the electrodes 302 and the headstage 304A. The pedestal 310 is secured, e.g., by screws, to the patient's 312 cranium 316, and extends outside the patient's 312 skin 318 through an incision (not labeled) in the skin 318. The wire bundle 308 is fed through the pedestal 310 to the headstage 304A mounted to the pedestal 310. The wireless module 306A is electrically coupled and mounted to the headstage 304A.

In general, the wire bundle 308 includes one wire for each electrode 302 included in subsystem 300A. For instance, if the electrodes 302 include an array of 96, 128, or 256 electrodes, the wire bundle 308 may respectively include 96, 128, or 256 separate wires.

In contrast, the interface between the headstage 304A and wireless module 306A may include fewer electrical connections implemented in complementary connectors of the headstage 304A and wireless module 306A. For instance, the interface between the headstage 304A and wireless module 306A may include seven electrical connections in some embodiments, including three connections for power (e.g., ground, positive supply voltage and negative supply voltage), two connections for a differential input clock from the wireless module 306A to the headstage 304A, and two connections for differential data output from the headstage 304A to the wireless module 306A.

Moreover, as shown in FIG. 3A, the wireless module 306A may include an electrical connection(s) 322 to a portable or non-portable power supply, such as a battery pack.

### B. Internal Implementation

FIG. 3B illustrates another embodiment of a collection, conditioning, and transmission subsystem 300B (hereinafter "subsystem 300B") including primarily internally implanted components such as may be implemented in the example operating environment 100 of FIG. 1 according to some embodiments. The subsystem 300B may correspond to the subsystems 106, 200 of FIGS. 1 and 2. Similar to the subsystem 300A of FIG. 3A, the subsystem 300B of FIG. 3B includes electrodes 302 and wire bundle 308. Additionally, the subsystem 300B includes an implantable headstage 304B electrically coupled to the electrodes 302 through wire bundle 308, and an implantable wireless module 306B coupled to the implantable headstage 304B through a second wire bundle 324.

As seen in FIG. 3B, the implantable headstage 304B is implanted subcutaneously within the patient 312. In particular, the implantable headstage 304B is implanted between the skin 318 and the cranium 316 of the patient 312.

As further seen in FIG. 3B, the implantable wireless module 306B is implanted within a cavity 326 of the patient 312. The cavity 326 may be the patient's 312 chest cavity in some embodiments, or other patient 312 cavity having sufficient space to accommodate the implantable wireless module 306B.

The subsystem 300B of FIG. 3B further includes an inductively rechargeable battery 328 implanted within the chest cavity 326 and electrically coupled to the implantable wireless module 306B. According to some embodiments, the battery 328 is the power source for the entire subsystem 300B. Generally, the battery 328 includes, or is electrically coupled to, an induction coil 329.

In the illustrated embodiment, the induction coil 329 is implanted near the ventral end of the patient's 312 dorsoventral axis. In other words, in the illustrated embodiment, the induction coil 329 is implanted towards or in front of the ventral side of the chest cavity 326. In particular, the induction coil 329 may be implanted between the ventral side of the patient's 312 rib cage, denoted in FIG. 3B at 330, and the patient's 312 skin 316. The positioning of the induction coil in front of the ventral side of the chest cavity 326 permits the patient 312 to easily position an external charger for charging the battery 328.

In operation, an inductive charger 332 that may optionally be included in the subsystem 300B can be employed to charge the battery 328. In particular, the inductive charger 332 is positioned external to the patient 312 in relatively close proximity to the induction coil 329 coupled to the battery 328, and operated to create an alternating electromagnetic field that penetrates through the patient's 312 skin 316 to the induction coil 329. The induction coil 329 then converts the alternating electromagnetic field to electrical current to charge the battery 328.

Accordingly, in some embodiments, the electrodes 302, implantable headstage 304B and implantable wireless module 306B are each completely implanted subcutaneously within the patient without being physically coupled to any components outside of the patient. As such, the electrodes 302, implantable headstage 304B, implantable wireless module 306B and physical connections between the electrodes 302, implantable headstage 304B and implantable wireless module 306B are completely enclosed within the patient 312. By completely enclosing the subsystem 300B within the patient 312, one or more incisions created in the patient's 312 skin 318 during installation of the subsystem 300B can be allowed to completely close and heal, thereby eliminating possible infection sites that exist in embodiments such as FIG. 3A where the incision (not labeled) through which the pedestal 310 extends necessarily remains open to accommodate the direct physical connection (e.g., the wire bundle 308) between the electrodes 302 and externally mounted headstage 304A.

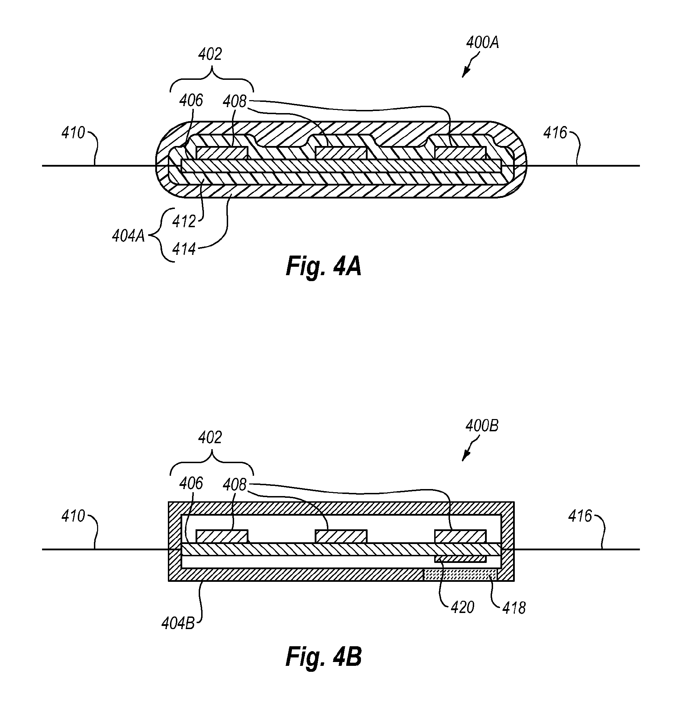

FIG. 4A illustrates a cross-sectional view of an implantable component 400A that generically represents an implantable headstage, an implantable wireless module, or other implantable component that may be implemented in one or more of the subsystems 106, 200, 300A, 300B of FIGS. 1-3B according to some embodiments. In general, the implantable component 400A includes a printed circuit board assembly ("PCBA") 402 and a bio-compatible housing 404A. More generally, the implantable component 404A may include one or more electronics encapsulated within bio-compatible housing 404A.

The PCBA 402 may include a printed circuit board ("PCB") 406 and a plurality of integrated circuits ("ICs") 408 attached to the PCB 406. Where the implantable component 400A is an implantable headstage and/or in other embodiments, the ICs 408 include, for example, an amplifier IC, one or more analog-to-digital converter ("ADCs") ICs, and a controller IC, aspects of which are disclosed in United States Patent Application No. 12/906,792, which was previously incorporated by reference.

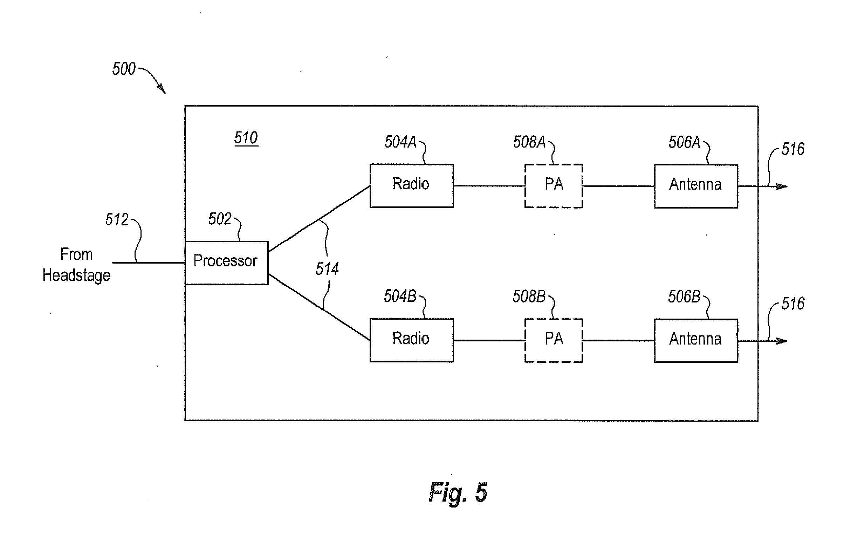

Where the implantable component 400A is an implantable wireless module and/or in other embodiments, the ICs 408 may include, for example, a processor IC, one or more radio ICs, and one or more post-amplifier ("PA") ICs, and the PCBA may further include one or more antennas (not shown) attached to the PCB 406. Additional details regarding ICs and other components that may be included in a wireless module according to some embodiments are provided below with respect to FIG. 5.

With continued reference to FIG. 4A, the bio-compatible housing 404A encapsulates the PCBA 402 and generally prevents direct interaction between the ICs 408 or other components of the PCBA 402 with surrounding tissue, blood, cerebrospinal fluid, or other fluids of a patient. Accordingly, the bio-compatible housing 404A may include one or more biomaterials, e.g., natural or synthetic material(s) that is (are) suitable for introduction into living tissue. For example, the bio-compatible housing 404A, in some embodiments, includes a polymer layer 412 substantially coating the PCBA 402 and a bio-compatible silicone layer 414 coating the polymer layer 412.

The polymer layer 412 may include Parylene or other suitable polymer. In general, Parylene includes derivatives of p-xylylene, such as, but not limited to, di-p-xylylene (also known as paracyclophane), Parylene N (hydrocarbon), Parylene C (one chlorine group per repeat unit), Parylene D (two chlorine groups per repeat unit), Parylene AF-4 (aliphatic fluorination 4 atoms), Parylene SF, Parylene HT, Parylene A (one amine per repeat unit), Parylene AM (one methylene amine group per repeat unit), Parylene VT-4 (fluorine atoms on the aromatic ring), or other suitable p-xylylene derivative.

The polymer layer 412 is generally configured to function as a moisture barrier and/or electrical insulator between the PCBA 402 and the bio-compatible silicone layer 414 and/or surrounding tissue of a patient. Accordingly, any suitable polymer material, including, but not limited to, Parylene, Para-xylene, polyimide, polyurethane, epoxy, or the like, can be implemented in the polymer layer 412. Alternately or additionally, any non-polymer material—such as, but not limited to, Silicon Nitride—having the appropriate characteristics to function as a moisture barrier and/or electrical insulator can be substituted for the polymer layer 412.

The layer 414 is generally configured for introduction into living tissue without causing any serious adverse affects, such as rejection by the body of the patient. While the layer 414 has been described as including bio-compatible silicone, any other suitable material(s) can be implemented in the layer 414. Examples of other suitable materials that can be used in the layer 414 include, but are not limited to, polymers, including Para-xylene, polyimide, polyurethane, epoxy, or the like.

Where the implantable component 400A is an implantable headstage, a first wire bundle 410 may be coupled to a corresponding plurality of electrodes and a second wire bundle 416 may be coupled to a corresponding wireless module, and the first and second wire bundles 410, 416 are configured to penetrate through the bio-compatible housing 404A and couple to the PCBA 406. Where the implantable component 400A is an implantable wireless module, the first wire bundle 410 may be coupled to a corresponding headstage and the second wire bundle 416 may be omitted or may be coupled to a battery or other power source.

FIG. 4B illustrates a cross-sectional view of an example of another implantable component 400B according to some embodiments. Similar to the implantable component 400A of FIG. 4A, the implantable component 400B generically represents an implantable headstage, an implantable wireless module, or other implantable component that may be implemented in one or more of the subsystems 106, 200, 300A, 300B of FIGS. 1-3B. The implantable component 400B is similar in some respects to the implantable component 400A of FIG. 4A and like reference numbers are used to designate like components.

In contrast to the implantable component 400A of FIG. 4A, the implantable component 400B of FIG. 4B includes a different bio-compatible housing 404B. In particular, the bio-compatible housing 404B may include titanium, a titanium alloy, stainless steel, other suitable biocompatible metal(s), biocompatible ceramic(s), or any combination thereof. Additionally, feed-throughs (not shown) may be provided in the bio-compatible housing 404B through which the first and optional second wire bundles 410, 416 electrically couple to the PCBA 402 encapsulated by bio-compatible housing 404B. The implantable component 400B of FIG. 4B in some embodiments is configured for use in chronic settings involving implantation of the implantable component 400B within a patient for more than, e.g., 30 days.

Where the implantable component 400B is an implantable wireless module, the bio-compatible housing 404B may include at least one region that is substantially transparent to the wireless signals emitted by the implantable wireless module. For instance, as shown in FIG. 4B, the bio-compatible housing 404B may include a signal-transparent region 418 generally aligned with a corresponding signal-emitting device 420 of the implantable component 400B. In some embodiments, the signal-emitting device 420 is an RF antenna, the signal-transparent region 418 includes ceramic, and the rest of the bio-compatible housing 404B includes titanium, a titanium alloy, ceramic, or other material that may be either opaque or transparent to RF signals emitted by the signal-emitting device 420.

### III. WIRELESS MODULE EMBODIMENTS

FIG. 5 is a functional block diagram of an embodiment of a wireless module 500 such as may be implemented in the subsystems 106, 200, 300A, 300B of FIGS. 1-3B. The wireless module 500 may correspond to one or more of the wireless modules 206, 306A, 306B of FIGS. 2-3B and/or may include a bio-compatible housing such as described with respect to FIGS. 4A-4B.

As shown in FIG. 5, the wireless module 500 includes a processor 502 or other control module such as a microprocessor, controller, microcontroller, or the like, and further includes a plurality of radio circuits 504A, 504B (collectively "radio circuits 504") and a plurality of antennas 506A, 506B (collectively "antennas 506"). Optionally, the wireless module 500 further includes a plurality of post-amplifier ("PA") circuits 508A, 508B (collectively "PA circuits 508").

The processor 502, radio circuits 504, PA circuits 508 and antennas 506 may be mounted to a PCB 510 or other carrier. Further, each of the processor 502, radio circuits 504 and PA circuits 508 may be implemented as separate ICs, or one or more of the processor 502, radio circuits 504 and PA circuits 508 may be implemented in one or more combined ICs.

The processor 502 is electrically coupled to a corresponding headstage via a serial link 512 that may be implemented as a differential signal pair or single-ended signal line. In some embodiments, digital data received over serial link 512 is packetized according to a first format implemented by the corresponding headstage. Packets according to the first format may include primarily data and a relatively minimal amount of overhead including, for instance, one or more of start bits, status bits, or cyclic redundancy check ("CRC") bits.

The processor 502 may be configured to re-format the digital data received over serial link 512 into a second format different than the first format that is suitable for wireless transmission. The second format may include packetized data with more overhead than the first format. The re-formatted data is redundantly provided by the processor 502 over a USB bus 514 to radio circuits 504 arranged in a multiple-input multiple-output ("MIMO") configuration.

The radio circuits 504 generate modulation currents representing the re-formatted data received from the processor 502 and the modulation currents are applied to antennas 506 to emit MIMO RF signals 516 representing the re-formatted data received from the processor 502.

In embodiments that include PA circuits 508, the PA circuits 508 amplify the modulation current provided by radio circuits 504. The gain of the PA circuits 508 may be controlled by the processor 502 to set the power (and thus the range) of the RF signals 516 emitted by antennas 506.

In some embodiments, the wireless module 500 is configured to implement a modified 802.11n protocol. According to these and other embodiments, the wireless module 500 may be configured to wirelessly transmit packetized data received from processor 502 substantially continuously without waiting to receive acknowledge packets from a corresponding receive subsystem indicating that the packetized data is being received by the receive subsystem. Alternately or additionally, the receive subsystem may be configured to wirelessly receive packetized data from the wireless module 500 substantially continuously without transmitting acknowledge packets to the wireless module 500.

Thus, wireless module 500 in some embodiments does not switch back and forth between a transmit mode and a receive mode as with some conventional wireless transmitters. Instead, the wireless module 500 may be configured to operate primarily or entirely in transmit mode. In these and other embodiments, the output data rate of the wireless module 500 may be between about 32 megabits per second ("Mb/s") and about 48 Mb/s. Alternately or additionally, the output data rate of the wireless module 500 may be less than 32 Mb/s or more than 48 Mb/s.

Although some embodiments of the wireless module 500 do not generally operate in a receive mode to receive acknowledge packets and thereby ensure packet delivery, packet delivery can be effectively ensured in some embodiments by selecting the power of the wireless module 500 based on a predetermined acceptable packet loss and a predetermined maximum transmission distance.

The predetermined acceptable packet loss generally depends on the particular application for which the wireless module 500 is being used. Some applications merely involve determining whether a neuron has fired, while other applications involve determining the shape of and classifying the neuron. Generally, a greater amount of packet loss can be tolerated in the former applications than in the latter applications.

The predetermined maximum transmission distance depends on the distance of a corresponding receive subsystem from the wireless module 500. Generally, however, the receive subsystem may be within several meters or less of the wireless module 500.

Accordingly, selecting the power of the wireless module 500 in some embodiments may include operating the wireless module 500 to emit a wireless data signal; measuring the packet loss at the predetermined maximum distance from the wireless module 500, reducing the power of the wireless module 500 to a "minimum" power at which the measured packet loss is about equal to the predetermined acceptable packet loss, and then slightly increasing the power of the wireless module 500 to an "operating" power above the minimum power to provide a margin of error.

FIG. 5 illustrates a wireless module 500 in which the wireless signal carrier is an RF signal. Those skilled in the art will understand, with the benefit of the present disclosure, various modifications that can be made to implement a wireless module that uses other wireless signal carriers, such as IR signals. In the case of an IR wireless signal carrier for instance, rather than radios 504 and antennas 506, the wireless module 500 may include one or more drivers coupled to one or more corresponding optical emitters (e.g., semiconductor lasers) configured to emit IR signals. By applying a modulation current generated by the one or more drivers to the one or more optical emitters, the optical emitters can emit an IR signal representative of data received from the processor 502.

### IV. EMBODIMENTS OF METHODS OF OPERATION

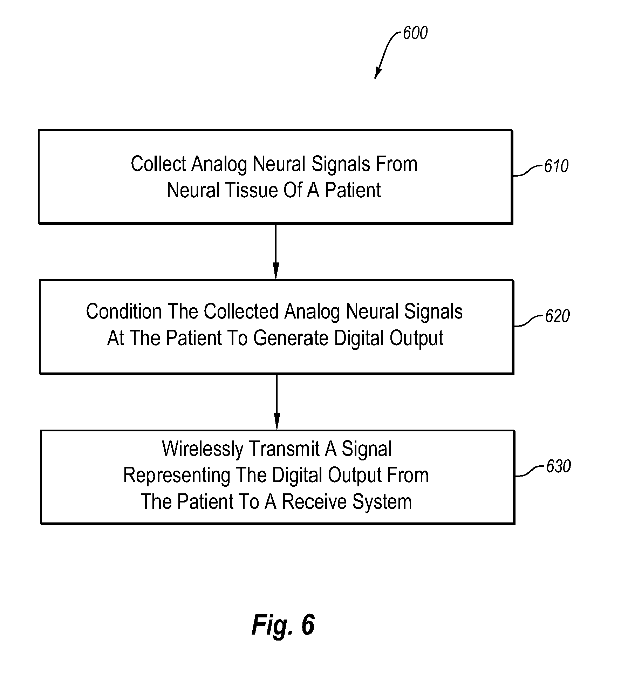

Turning next to FIG. 6, a method of operation is described according to some embodiments. One skilled in the art will appreciate that, for processes and methods disclosed herein, the acts performed in the processes and methods may be implemented in differing order than disclosed herein. Furthermore, the outlined acts and operations are only provided as examples, and some of the acts and operations may be optional, combined into fewer acts and operations, or expanded into additional acts and operations without detracting from the essence of the disclosed embodiments.

FIG. 6 is a flowchart of an embodiment of a method 600 of collecting and conditioning analog neural signals. The method 600 of FIG. 6 is implemented in some embodiments by a neuralphysiological data acquisition system, such as the system 104 of FIG. 1, including a plurality of electrodes, such as the electrodes 202 of FIG. 2, an implantable or externally mounted headstage, such as the headstages 204, 304A, 304B of FIGS. 2-3B, an implantable or externally mounted wireless module, such as the wireless modules 206, 306A, 306B, 500 of FIGS. 2-3B and 5, and optionally a receive subsystem, such as the receive subsystem 108 of FIG. 1.

The method 600 begins, in some embodiments, by collecting 610 analog neural signals from neural tissue of a patient. The act 610 of collecting analog neural signals is performed, in some embodiments, by electrodes included in a neuralphysiological data acquisition system.

The method 600 additionally includes conditioning 620 the collected analog neural signals at the patient to generate a digital output representing the collected analog neural signals. The act 620 of conditioning the collected analog neural signals at the patient to generate a digital output is performed, in some embodiments, by a headstage. Further, conditioning 620 the collected analog neural signals at the patient may include conditioning the collected analog neural signals within the patient in an implantable headstage, or externally to the patient in an externally mounted headstage.

According to some embodiments, conditioning 620 the collected analog signals includes amplifying the collected analog neural signals, filtering the amplified analog neural signals, multiplexing the filtered analog neural signals, digitizing the multiplexed analog neural signals to generate digital neural signals, and packetizing the digital neural signals for inclusion in the digital output.

The method 600 additionally includes wirelessly transmitting 630 a signal representing the digital output from the patient to a receive subsystem including a wireless receiver. The act 630 of wirelessly transmitting the signal representing the digital output to the receive subsystem may be performed by a wireless module. According to some embodiments, the signal representing the digital output is continuously wirelessly transmitted to the receive subsystem without waiting to receive acknowledge packets from the receive subsystem indicating that the signal is being received by the receive subsystem.

Alternately or additionally, wirelessly transmitting the signal representing the digital output includes re-formatting the digital output from a first format to a second format and driving a plurality of antennas arranged in a MIMO configuration using the re-formatted digital output in the second format.

Other acts and operations not shown in FIG. 6 or described above can optionally be included in the method 600. As an example, the method 600 may further include receiving the signal representing the digital output at the receive subsystem and performing an action according to the signal. Optionally, performing an action according to the signal may include one of: delivering a drug to a patient in response to identifying a predetermined pattern in the signal, the predetermined pattern being indicative of an oncoming biological event in the patient, the oncoming biological event being preventable by the drug; generating an alarm in response to identifying a predetermined pattern in the signal, the predetermined pattern being indicative of an oncoming biological event; driving a prosthetic limb to operate in accordance with the signal; or synthesizing speech corresponding to a word represented by the signal.

As another example, the method 600 may further include receiving the signal representing the digital output at the receive subsystem and extracting information about a brain function of the patient from the signal representing the digital output. The act of extracting information about a brain function of the patient from the signal representing the digital output may be performed by an external processing system, for example.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A neuralphysiological data acquisition system, comprising:
a plurality of electrodes configured to be implanted subcutaneously within neural tissue of a patient and to collect analog neural data from the patient;
a headstage coupled to the plurality of electrodes and configured to receive and convert the analog neural signals to digital output; and
a wireless module coupled to the headstage and configured to wirelessly transmit a signal representing the digital output to a receive subsystem including a wireless receiver.

2. The neuralphysiological data acquisition system of claim 1 wherein the headstage and wireless module each comprises a bio-compatible housing and each is configured to be implanted subcutaneously within the patient.

3. The neuralphysiological data acquisition system of claim 2 wherein the plurality of electrodes, headstage, and wireless module are each configured to be completely implanted subcutaneously within the patient without being physically coupled to any components outside of the patient such that the plurality of electrodes, headstage, wireless module and physical connections between the plurality of electrodes, headstage, and wireless module can be completely enclosed within the patient.

4. The neuralphysiological data acquisition system of claim 2 wherein the bio-compatible housings of both the headstage and the wireless module include titanium, a titanium alloy, or combinations thereof.

5. The neuralphysiological data acquisition system of claim 4 wherein the bio-compatible housing of the wireless module comprises a window that is substantially transparent to the signal representing the digital output.

6. The neuralphysiological data acquisition system of claim 1 wherein the wireless module comprises a plurality of radios arranged in a multiple-input and multiple-output ("MIMO") configuration.

7. The neuralphysiological data acquisition system of claim 1 wherein the wireless module is configured to wirelessly transmit packetized data substantially continuously without waiting to receive acknowledge packets from the receive subsystem indicating that the packetized data is being received by the receive subsystem.

8. The neuralphysiological data acquisition system of claim 1 wherein power consumption of the neuralphysiological data acquisition system is about four watts.

9. The neuralphysiological data acquisition system of claim 1 wherein the power source comprises an inductively rechargeable battery configured to be implanted subcutaneously within the patient.

10. The neuralphysiological data acquisition system of claim 1, further comprising the receive subsystem, wherein the receive subsystem comprises a drug delivery device configured to deliver a drug to the patient in response to the drug delivery device identifying a predetermined pattern in the signal received from the wireless module.

11. The neuralphysiological data acquisition system of claim 1, further comprising the receive subsystem, wherein the receive subsystem comprises at least one of (1) a data storage device configured to store data representing the signal received from the wireless module; (2) a notification device configured to generate an alarm in response to the notification device identifying a predetermined pattern in the signal received from the wireless module; (3) a prosthetic limb configured to operate in accordance with the signal received from the wireless module; or (4) a voice synthesizer configured to synthesize speech corresponding to a word represented by the signal received from the wireless module.

12. The neuralphysiological data acquisition system of claim 1 wherein the wireless module comprises:
a processor configured to receive the digital output from the headstage and further configured to convert the digital output from a first format to a digital signal having a second format;
a plurality of radios coupled to the processor, each of the plurality of radios configured to receive the digital signal having the second format and to generate respective modulation signals representing the digital signal having the second format; and
a plurality of antennas, each of the plurality of antennas coupled to a respective one of the plurality of radios to receive a corresponding one of the modulation signals and configured to emit a radio frequency ("RF") signal representing the corresponding one of the modulation signals.

13. A method of collecting and conditioning neural signals, the method comprising:
collecting analog neural signals from neural tissue of a patient;
conditioning the collected analog neural signals at the patient to generate a digital output representing the collected analog neural signals; and
wirelessly transmitting a signal representing the digital output from the patient to a receive subsystem including a wireless receiver.

14. The method of claim 13 wherein the signal is continuously wirelessly transmitted without waiting to receive acknowledge packets from the receive subsystem indicating that the signal is being received by the receive subsystem.

15. The method of claim 13, further comprising:
receiving the signal representing the digital output at the receive subsystem; and
performing an action according to the signal, wherein performing an action according to the signal comprises at least one of:
delivering a drug to a patient in response to identifying a predetermined pattern in the signal, the predetermined pattern being indicative of an oncoming biological event in the patient, the oncoming biological event being preventable by the drug;
generating an alarm in response to identifying a predetermined pattern in the signal, the predetermined pattern being indicative of an oncoming biological event;
driving a prosthetic limb to operate in accordance with the signal; or
synthesizing speech corresponding to a word represented by the signal.
